# EUROPÄISCHE PATENTSCHRIFT

(11) **EP 0 844 828 B1**
(45) Veröffentlichungstag und Bekanntmachung des Hinweises auf die Patenterteilung: **02.11.2006**
(21) Anmeldenummer: 96927633.6
(22) Anmeldetag: 30.07.1996
(51) Int. Cl.: A01N 61/00, A01N 37/50

(54) **VERFAHREN ZUR BEKÄMPFUNG VON FISCHMYCOSEN UND EINZELLIGEN EKTOPARASITEN**
METHOD FOR COMBATTING FISH MYCOSES AND ONE-CELLED ECTOPARASITES
PROCEDE DE LUTTE CONTRE DES MYCOSES DE POISSONS ET DES ECTOPARASITES MONOCELLULAIRES

(30) Priorität: 17.08.1995 DE 19530175
(43) Veröffentlichungstag der Anmeldung: 03.06.1998
(73) Patentinhaber: BASF Aktiengesellschaft, 67056 Ludwigshafen (DE)
(72) Erfinder: DOHMEN, Gerhard, Peter, D-69469 Weinheim (DE); KÜNAST, Christoph, D-67166 Otterstadt (DE); MUNK, Reinhart, D-67117 Limburgerhof (DE); ROTHHAAS, Gerhard, D-67117 Limburgerhof (DE)
(74) Vertreter: Münch, Volker
(86) Internationale Anmeldenummer: PCT/EP1996/003354
(87) Internationale Veröffentlichungsnummer: WO 1997/006690

(56) Entgegenhaltungen:
- EP-A- 0 167 710
- EP-A- 0 253 213
- EP-A- 0 279 218
- US-A- 3 152 953
- NATURAL PRODUCT REPORTS, 1993, Seiten 565-74, XP000564143 J.M.CLOUGH: "The Strobilurins, Oudemansins, and Myxothiazols, Fungicidal Derivatives of beta-Methoxyacrylic-Acid" in der Anmeldung erwähnt
- BIOCHEMICAL SOCIETY TRANSACTIONS, Bd. 22, 1993, Seite 63S XP000563596 F. ROEHL ET AL.: "Species dependence of mitochondrial respiration inhibition by strobilurin analogues" in der Anmeldung erwähnt
- BIOCHEMICAL SOCIETY TRANSACTIONS , Bd. 22, 1993, Seite 65S XP000563594 H.KÖHLE ET AL.: "Biokinetic properties of BAS 490 F and some related compounds"
- CHEMICAL ABSTRACTS, vol. 96, no. 13, 29.März 1982 Columbus, Ohio, US; abstract no. 99524r, W.F.BECKER ET AL.: "Oudemansin, strobilurin A, strobilurin B, and myxothiazol: new inhibitors of the bc1 segment of the respiratory chain with an E-beta-methoxyacrylate system as common structural element" XP002021380 & FEBS LETT., Bd. 132, Nr. 2, 1981, Seiten 329-33,

## Beschreibung

Die vorliegende Erfindung betrifft die Verwendung einer Verbindung der Formel I 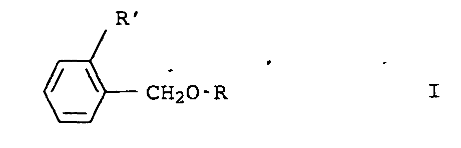 in der die Substituenten die folgende Bedeutung haben:
- R': C[CO₂CH₃]=NOCH₃;
- R: 2-CH₃-C₆H₄; 2,5-(CH₃)₂-C₆H₃; 2,4-(CH₃)₂-C₆H₃; 2,3,5-(CH₃)₃-C₆H₂; 2-Cl, 5-CH₃-C₆H₃ oder 2-CH₃, 4-C[CH₃]=NOCH₃-C₆H₃;
zur Herstellung eines zur Bekämpfung von Fischmycosen und einzelligen Ektoparasiten von Fischen geeigneten Mittels.

Bisher sind nur wenige Substanzen bekannt, die zur Bekämpfung von Fischmycosen und einzelligen Ektoparasiten geeignet sind.

Das bisher vorwiegend genutzte Präparat "Malachitgrün" mußte wegen seiner Teratogenität [*vgl. Meyer et al., Transactions of the American Fisheries Society, 112, 818-824*] und Verdacht auf Cancerogenität vom Markt genommen werden. Andere Produkte (z.B. Formalin, Kalium-Permanganat, Kochsalz) sind nur wenig wirksam und haben zum Teil unerwünscht starke Einflüsse auf die Umwelt. Zu den bisher gegebenen Möglichkeiten zur Bekämpfung von Fischparasiten siehe W. Schäperclaus, Fischkrankheiten (2. Teil), 4. Auflage, Akademie-Verlag Berlin, 1979 und H.-H. Reichenbach-Klinke, Reichenbach-Kline's Fish Pathology, T.S.H. Publications Inc. Ltd., 1973.

Wirkstoffe, die die mitochondriale Atmungskette auf der Stufe des b/c₁-Komplexes hemmen, sind aus der Literatur als Fungizide bekannt [vgl. Dechema-Monographien Bd. 129, 27-38, VCH Verlagsgemeinschaft Weinheim 1993; Natural Product Reports 1993, 565-574; Biochem. Soc. Trans. 22, 63S und 65S (1993); FEBS Lett., Bd. 132, 329 (1981)]. Dazu gehört auch Antimycin, das als wirksames Fischgift bekannt ist [vgl. US 3,152,953].

In EP-A 167 710 werden zur Bekämpfung von Fischmycosen Benzimidazolderivate vorgeschlagen. Aus EP-A 279 218 sind Triazintrione zur Bekämpfung von Fischparasiten bekannt.

Vertreter der Wirkstoffe der Formel I mit fungizider und zum Teil auch bioregulatorischer Wirkung werden in den folgenden Schriften beschrieben:
EP-A 253 213, EP-A 386 561 und EP-A 400 417.

Der vorliegenden Erfindung lagen daher Mittel zur Bekämpfung von Fischmycosen und einzelligen Ektoparasiten auf Fischen als Aufgabe zugrunde, die sich bei guter Wirksamkeit nicht in unerwünschtem Maße im Fisch anreichern und keine unakzeptablen Auswirkungen auf die Lebensgemeinschaften haben.

Demgemäß wurde die eingangs definierte Verwendung gefunden.

Für die Bereitstellung der erfindungsgemäßen Mittel eignen sich die in den eingangs genannten Schriften beschriebenen Wirkstoffe, sofern die in Tabelle A genannten Substituentenbedeutungen vorliegen:

**Tabelle A**

| Verbindungen der Formel I, in denen Q Phenyl bedeutet, R' für -C(CO₂CH₃)=NOCH₃ steht, R für ggf. subst. (Het)aryl-oxymethylen steht, wobei die ggf. subst. (Het)arylgruppe die folgende Bedeutung hat | | |
|---|---|---|
| Nr. | ggf. subst. (Het)aryl | Literatur |
| A-1 | 2-CH₃-C₆H₄ | EP-A 253 213 |
| A-2 | 2,5-(CH₃)-₂-C₆H₃ | EP-A 400 417 |
| A-3 | 2,4-(CH₃)₂-C₆H₃ | EP-A 400 417 |
| A-4 | 2,3,5-(CH₃)₃-C₆H₂ | EP-A 400 417 |
| A-5 | 2-C1, S-CH₃-C₆H₃ | EP-A 400 417 |
| A-6 | 2-CH₃, 4-C[CH₃]=NOCH₃-C₆H₃ | EP-A 386 561 |

Es ist nach den bisherigen Erkenntnissen davon auszugehen, daß der erfindungsgemäße Effekt der Bekämpfung von Fischmycosen und einzelligen Ektoparasiten bei Fischen bei allen Fischpopulationen einsetzbar ist, insbesondere in Zuchtteichen, Zuchttanks und Aquarien, natürlichen Sportfisch-Gewässern und marinen Fischzuchten.

Die erfindungsgemäßen Mittel eignen sich besonders zur Behandlung von Cypriniden, Percidae und Salmoniden sowie anderen Zucht- und Zierfischen.

Insbesondere eignen sich die erfindungsgemäßen Mittel zur Bekämpfung von Branchiomykose und Saprolegniose bei Fischen, sowie deren Ei-, Larven- und Jungstadien.

Die erfindungsgemäßen Mittel eignen sich weiterhin zur Bekämpfung von insbesondere einzelligen Ektoparasiten auf Fischen, beispielhaft seien aufgeführt:
Ichthyophthirius sp., Chilodonella sp., Trichodina sp. und Ichthyobodo.

Das durch solche Parasiten hervorgerufene Krankheitsbild der Costiose kann durch die erfindungsgemäßen Wirkstoffe der Formel I bekämpft werden.

Die Aufwandmengen an Verbindungen I liegen je nach der Art bzw. Entwicklungsstadium der Fische bei 0.1 µg/l bis 5 mg/l, vorzugsweise 0.3 µg/l bis 3 mg/l, insbesondere 1 µg/l bis 1 mg/l, besonders bevorzugt 5 µg/l bis 500 µg/l. Höhere Konzentrationen sind im allgemeinen nicht erforderlich, können aber - je nach Art der Verbindung besonders in künstlichen Systemen (z.B. Zuchttanks oder Aquarien) bei der Behandlung von Ei-, Larven- und Jungstadien nützlich sein.

Die Verbindungen I können direkt angewendet werden oder vor der Anwendung mit üblichen inerten Trägerstoffen gemischt werden. Grundsätzlich eignen sich als inerte Trägerstoffe solche Substanzen, die eine homogene Verteilung des Wirkstoffs im Wasser erleichtern bzw. gewährleisten.

Die Mittel werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl-oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, LöB, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-Volumeverfahren (ULV) ausgebracht werden, wobei sogar der Wirksoff ohne Zusätze verwendet werden kann.

Die Wirkstoffe werden normalerweise in einer Reinheit von 90 % bis 100 %, vorzugsweise 95 % bis 100 % (nach NMR-Spektrum) eingesetzt.

Die Wirkung zur Bekämpfung von Fischmycosen ließ sich durch die folgenden Versuche zeigen.

Bei Untersuchungen in Kleingewässern zur ökotoxikologischen Abschätzung von fungiziden Pflanzenschutzmittel wurden auch Fische getestet. Gruppen von jeweils 7 Tieren der Art Cyprinus carpio wurden in vier Behälter befüllt mit 6,5 m³ eines natürlichen betamesosaproben Gewässers und natürlichem Sediment unter Feilandbedingungen gehalten. Ein Behälter diente als Kontrolle, die anderen wurden mit verschiedenen Konzentrationen des Wirkstoffs A-1 behandelt: 0; 1,3 µg/l; 6,7 µg/l und 33,3 µg/l.

Die Wirkstoffe wurden dabei als 50 Gew.-% in Wasser dispergierbares Granulat der folgenden Zusammensetzung verwendet:

| | |
|---|---|
| 50 Gew.-% | Wirkstoff |
| 10 Gew.-% | Träger (Ammoniumsulfat) |
| 1 Gew.-% | Pluronic® PE 6800 (Addukt aus Ethylenoxid und Polypropylenglycol, Dispergiermittel) |
| 0,84 Gew.-% | Antischaummittel (33%-ige wäßrige Emulsion von Polydimethylsiloxan) |

auf 100 Gew.-% ergänzt mit einer 2:1 Mischung aus
- Natriumlignosulfonat und
- dem Natriumsalz des Formaldehydkondensats von Naphthalinsulfonsäure.

Die zufallsverteilten Fische wurden am 25.04.94 eingesetzt. Am 03.05.94 wurde eine Fungizidbehandlung der Becken durchgeführt. Im weiteren Verlauf des Versuches erkrankten und verendeten an Pilzbefall in dem Kontrollbecken (keine Fungizidbehandlung) 4 von 7 Fischen, in der kleinsten Konzentration kein Fisch (jedoch 2 vor der Behandlung) und in den beiden höheren Konzentrationen ebenfalls keiner der Fische.

Das heißt, während in der Kontrolle gut die Hälfte der Fische mit Pilzen befallen wurden und daran auch verendeten, trat in den mit A-1 behandelten Becken kein Pilzbefall auf. Alle Fische blieben gesund.

Am Ende des Versuchs nach mehreren Behandlungen wurde die Größenentwicklung der Fische sowie ihr allgemeiner Gesundheitszustand überprüft. Dabei fanden sich keinerlei nagativen Einflüsse des Fungizides auf die Fische.

## Patentansprüche

1. Verwendung einer Verbindung der Formel I, 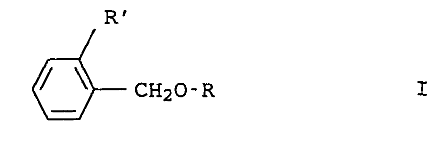 in der die Substituenten die folgende Bedeutung haben:
R' C[CO₂CH₃] =NOCH₃;
R 2-CH₃-C₆H₄; 2,5-(CH₃)₂-C₆H₃; 2,4-(CH₃)₂-C₆H₃ ; 2,3,5-(CH₃)₃-C₆H₂; 2-Cl, 5-CH₃-C₆H₃ oder 2-CH₃, 4-C[CH₃]=NOCH₃-C₆H₃;
zur Herstellung eines zur Bekämpfung von Fischmycosen und einzelligen Ektoparasiten von Fischen geeigneten Mittels.

2. Verwendung der Verbindung 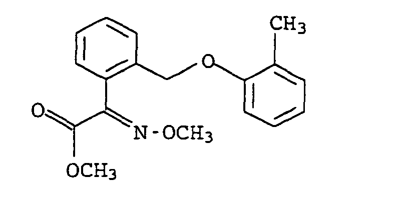 nach Anspruch 1.

## Claims

1. The use of a compound of the formula I 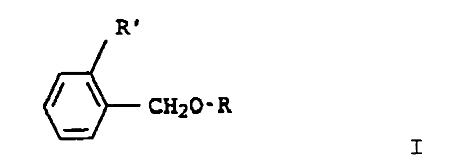 in which the substituents have the following meaning:
R' C [CO₂CH₃] =NOCH₃;
R 2-CH₃-C₆H₄; 2,5-(CH₃)₂-C₆H₃; 2,4-(CH₃)₂-C₆H₃; 2,3,5-(CH₃)₃-C₆H₂; 2-Cl, 5-CH₃-C₆H₃ or 2-CH₃, 4-C [CH₃] =NOCH₃-C₆H₃;
for the production of a composition suitable for controlling fish mycoses and single-cell ectoparasites of fish.

2. The use of the compound 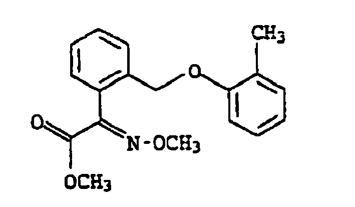 according to Claim 1.

## Revendications

1. Utilisation d'un composé de la formule I 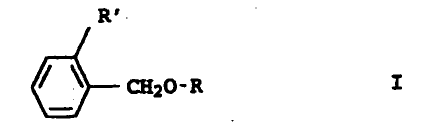 dans laquelle les substituants ont les significations suivantes :
R' C[CO₂CH₃] =NOCH₃,
R 2-CH₃-C₆H₄, 2,5-(CH₃)₂-C₆H₃, 2,4-(CH₃)₂-C₆H₃, 2,3,5-(CH₃)₃-C₆H₂, 2-Cl, 5-CH₃-C₆H₃ ou 2-CH₃, 4 -C [CH₃] =NOCH₃-C6H₃,
pour la préparation d'un agent approprié pour la lutte contre des mycoses des poissons et d'ectoparasites monocellulaires des poissons.

2. Utilisation du composé 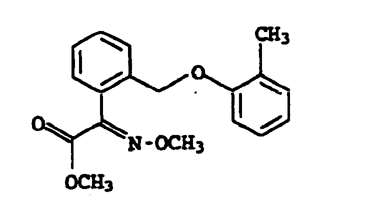 selon la revendication 1.
